# EUROPÄISCHE PATENTSCHRIFT

(11) **EP 0 834 076 B1**
(45) Veröffentlichungstag und Bekanntmachung des Hinweises auf die Patenterteilung: **08.12.1999**
(21) Anmeldenummer: 96922841.0
(22) Anmeldetag: 19.06.1996
(51) Int. Cl.: G01N 33/566, G01N 33/76, G01N 33/564

(54) **VERFAHREN ZUR BESTIMMUNG VON ANTI-TSH-REZEPTOR-AUTOANTIKÖRPERN**
PROCESS FOR DETERMINING AUTO-ANTIBODIES AGAINST ANTI-TSH-RECEPTOR AUTO-ANTIBODIES
PROCEDE DE QUANTIFICATION D'AUTO-ANTICORPS DIRIGES CONTRE LES RECEPTEURS DE TSH

(30) Priorität: 19.06.1995 DE 19522171
(43) Veröffentlichungstag der Anmeldung: 08.04.1998
(73) Patentinhaber: B.R.A.H.M.S DIAGNOSTICA GMBH, 12099 Berlin (DE)
(72) Erfinder: BERGMANN, Andreas, D-12351 Berlin (DE)
(74) Vertreter: Andrae, Steffen, Dr.
(86) Internationale Anmeldenummer: EP9602649
(87) Internationale Veröffentlichungsnummer: WO9700447

(56) Entgegenhaltungen:
- WO-A-95/06258

## Beschreibung

Die vorliegende Erfindung betrifft Verfahren zur klinischen Diagnose von Schilddrüsen-Autoimmunerkrankungen, und insbesondere ein verbessertes Verfahren zur Bestimmung von Anti-TSH-Rezeptor-Autoantikörpern in einer humanen Serum- oder Plasmaprobe mit Hilfe eines sogenannten Rezeptor-Assays.

Im Rahmen der Diagnose von Schilddrüsenerkrankungen hat die Bestimmung von Autoantikörpern gegen den TSH-Rezeptor ("Anti-TSH-Rezeptor-Autoantikörpern") erhebliche Bedeutung. Der TSH-Rezeptor spielt im Rahmen der Regulierung der Funktion der Schilddrüse eine zentrale Rolle. Durch Bindung des von der Hypophyse ausgeschütteten Hormons TSH (Thyrotropin) an den in der Schilddrüsenmembran verankerten TSH-Rezeptor wird nämlich die Bildung und Ausschüttung des wichtigsten Schilddrüsenhormons Thyroxin (T4) stimuliert.

An diesen TSH-Rezeptor können jedoch auch bei bestimmten Krankheitszuständen gebildete Autoantikörper binden, die nachfolgend in ihrer Gesamtheit als Anti-TSH-Rezeptor-Autoantikörper bezeichnet werden. Je nach Art dieser Autoantikörper kann es entweder zur Abschirmung der Bindung der TSH-Moleküle an den TSH-Rezeptor und damit zu einer Inhibierung der Bildung und Ausschüttung von Thyroxin kommen oder im Gegenteil dazu, daß dieses Schilddrüsenhormon unkontrolliert ausgeschüttet wird, weil die Anti-TSH-Rezeptor-Autoantikörper bei ihrer Bindung an den TSH-Rezeptor die Wirkung des TSH simulieren und die Synthese und Ausschüttung von Schilddrüsenhormonen stimulieren. Der im letzteren Falle resultierende Schilddrüsenhormonüberschuß äußert sich u.a. als eine Hyperthyreose vom Typ Morbus Basedow.

Die Bedeutung von Autoantikörpern gegen den TSH-Rezeptor wird in zahlreichen wissenschaftlichen Veröffentlichungen ausführlich diskutiert, wobei stellvertretend auf den Übersichtsartikel von Bernard Rees Smith et al., "Autoantibodies to the Thyrotropin Receptor", in: Endocrine Reviews, Vol. 9, No.1, 1988, S. 106 bis 121 verwiesen werden kann.

Wegen der Wichtigkeit des Nachweises von Anti-TSH-Rezeptor-Autoantikörpern in der klinischen Praxis werden von verschiedenen Diagnostikafirmen derartige Bestimmungsverfahren in Form von Kits angeboten. Alle derartigen gegenwärtig kommerziell angebotenen Verfahren zur Bestimmung von Anti-TSH-Rezeptor-Autoantikörpern arbeiten dabei nach dem Prinzip des sogenannten Radiorezeptor-Assays (RRA), bei dem man zur Bestimmung der gesuchten Autoantikörper in einer Serum- oder gegebenenfalls auch Plasmaprobe so vorgeht, daß man die zu gegebenfalls in einer Serum- oder Plasmaprobe vorhandenen Autoantikörper mit einem zugesetzten ¹²⁵I-markierten TSH-Präparat um die Bindungsstellen eines ebenfalls zugesetzten TSH-Rezeptors konkurrieren läßt, der durch Extraktion und Solubilisierung aus einem humanen oder tierischen Schilddrüsenmaterial, in der Regel aus Schilddrüsenmaterial vom Schwein, gewonnen wurde.

Alle Bestimmungsverfahren werden dabei nach ein und demselben Inkubationsschema durchgeführt, das im wesentlichen die folgenden Schritte umfaßt:
1. Pipettieren der Probe (des Patientenserums) in ein Teströhrchen,
2. Zugeben des solubilisierten TSH-Rezeptors in der für den jeweiligen Kit vorgesehenen Form, und Durchmischen,
3. Vorinkubieren der gebildeten Reaktionsmischung, die die Bestandteile der Probe sowie den TSH-Rezeptor enthält, für einen Zeitraum von in der Regel 15 bis 20 Minuten bei Raumtemperatur,
4. danach Zugeben des markierten TSH-Präparats als Tracer,
5. Inkubieren der nunmehr alle Testbestandteile enthaltenden Reaktionsmischung für einen Zeitraum in der Größenordnung von zwei Stunden ± 30 Minuten bei Raumtemperatur,
6. Zugeben eines Fällungsreagenz, in der Regel von Polyethylenglykol (PEG) zur Ausfällung des TSH-Rezeptors mit den daran gebundenen Anteilen des Tracers sowie ggf. der Autoantikörper,
7. Zentrifugieren und Abtrennen des flüssigen Überstands von der festen Phase sowie
8. Messen der Radioaktivität in der festen Phase.

Für die Auswertung der Meßergebnisse werden Eichkurven verwendet, die genau nach dem gleichen Verfahren erstellt werden, nur daß man anstelle der Serumproben die dem Kit beigefügten Standards bzw. Kontrollen einsetzt. Letztere können als "Proben" bekannter Zusammensetzung angesehen werden.

Wenn im Rahmen der vorliegenden Patentanmeldung von "Probe" gesprochen wird, ist somit nicht nur die aus einem Patienten gewonnene Serum- oder Plasmaprobe gemeint, sondern alles im Hinblick auf die Probe Gesagte gilt genauso für die Messung der Standardlösungen und Kontrollproben.

Daß nach dem gegenwärtigen Stand der Technik bei Radiorezeptor-Assays zur Bestimmung von Anti-TSH-Rezeptor-Autoantikörpern ausnahmslos nach der beschriebenen Arbeitsweise gearbeitet wird, läßt sich anhand der Arbeitsanweisungen aller diesseits bekannten einschlägigen Testkits belegen, nämlich:
Arbeitsanleitung TRAK-Assay® der Fa. B.R.A.H.M.S Diagnostica GmbH, Berlin, Januar 1995, insbesondere Meßprinzip gemäß den inneren Umschlagseiten sowie Abschnitt "Testdurchführung";
Arbeitsanleitung TSH REZAK® der Fa. Medipan Diagnostika Entwicklungs- und Vertriebs GmbH, Selchow, Abschnitt "Testdurchführung", (Stand 1.12.1994);
Gebrauchsinformation THYBIA-Assay der Fa. Byk-Sangtec Diagnostica GmbH & Co. KG, Dietzenbach, 1993, Abschnitt "Testablauf";
Herstellerinformation TSH RECEPTOR ANTIBODY (TRAb) KIT der Fa. KRONUS®, San Clemente, USA, 1993, insbesondere S.6, "Assay Protocol";
Herstellerinformation "Thyrotropin Receptor Autoantibodies", der Fa. Gesellschaft für Immunchemie und Immunbiologie mbH, Hamburg, Januar 1993, insbesondere Abschnitt 6;
Herstellerinformation TBIAb RRA der Fa. BIOCODE s.a., Sclessin, Belgien, April 1991, insbesondere Abschnitte 4.2 und 8.2;
Japanische Gebrauchsanleitung "TRAb" der Fa. RSR Limited, Cardiff, UK, 1994, insbesondere Abschnitt 2. Ziffer 4;
Herstellerinformation ANTI R-TSH der Fa. Immunotech, Frankreich, insbesondere Abschnitt 7. SCHEMA OPERATOIRE DU DOSAGE;
Herstellerinformation TRAB ¹²⁵I der Fa. Techland, Sept. 1992, insbesondere S.5 und 8.

Bei allen beschriebenen Verfahren wird es als erforderlich angesehen, die Reaktionsmischung zur Durchführung der eigentlichen Konkurrenz-Meßreaktion zweistufig zu bereiten, nämlich durch Zusammengeben der TSH-Rezeptor-Präparation mit der Probe und eine Vorinkubation der einen Teil des Reaktionssystems enthaltenden Reaktionslösung als erste Stufe, um eine Vorreaktion des TSH-Rezeptors mit den gesuchten Autoantikörpern zu ermöglichen, sowie, als zweite Stufe, durch zeitlich verzögerte Zugabe des als Tracer zugesetzten konkurrierenden TSH-Präparats nach Abschluß der Vorinkubation.

Nachdem auch der Tracer zugesetzt wurde, wird noch einmal für einen längeren Zeitraum, in der Regel von 2h bei Raumtemperatur oder von 1h bei 37 °C, eine Reaktionsmischung inkubiert, die nunmehr alle Umsetzungspartner enthält.

Eine Vorinkubation der genannten Art wird im gesamten Stand der Technik durchgeführt, wobei beispielsweise auf die folgenden Literaturstellen verwiesen werden kann:
S. Qasim Mehdi et al., in: Biochem. J. (1975) 145, S. 105-111, insbesondere S. 107, Abschnitt "Standard procedure for the assay of LATS in human sera or IgG";
H. Schleusener et al., J.Endocrinol.Invest. 2: S.155-161, (1978);
J. Haberman, C.R. Pickardt und P.C. Scriba, Acta Endocrinol. (Copenhagen) Suppl. 234, 26 (1980);
Bernard Rees Smith and Reginald Hall, "Measurement of Thyrotropin Receptor Antibodies" in: Methods in Enzymology, Vol. 74 (1981), S. 405-420, insbesondere S. 415, "Assay Procedure", S. 417, Zeilen 1-5 des Textes von unten;
Kay Southgate et al., in Clinical Endocrinology (1984), 20, S. 539-548, insbesondere S. 541, Abschnitt "Assay procedure for unextracted serum";
Haruo Tamaki et al., in: J.Clin.Lab.Immunol. (1986), 20, S.1-6; insbesondere S.2, Abschnitt "Assay Procedure for Unextracted Sera";
S. Costagliola et al., "Binding Assay for Thyrotropin Receptor Autoantibodies Using the Recombinant Receptor Protein" in: Journal of Clinical Endocrinology and Metabolism, Vol. 78, No.6, (1992) S. 1540-1544, insbesondere S. 1541, Abschnitt "Binding Assay for autoantibody determinations";
Manjula K. Gupta in: Clin. Biochem., Vol. 25, 1992, S. 193-199, insbesondere S. 194, linke Spalte.

Die angeführten Literaturstellen decken einen Zeitraum von 20 Jahren ab, wobei gemäß den älteren Literaturstellen die Anti-TSH-Rezeptor-Autoantikörper auch als LATS (long-acting thyroid stimulator) oder TBII (TSH-binding inhibitor immunoglobulin) bezeichnet werden. Eine in der Literatur zu findende Abkürzung ist auch TRAb (thyrotropin (TSH)-receptor antibodies).

Die frühesten einschlägigen Verfahren arbeiteten stets mit Schilddrüsenmembranen als Rezeptor-Präparation sowie vorzugsweise mit isolierten Immunglobulin-Fraktionen als Probenmaterial. Dabei wird schon in der ältesten der oben aufgeführten Arbeiten eine Vorinkubation von drei Stunden bei 23 bis 25 °C durchgeführt. In der o.g. Literaturstelle J. Haberman, C.R. Pickardt und P.C. Scriba, Acta Endocrinol. Copenhagen) Suppl. 234, 26 (1980) wird dann ausdrücklich auf die Bedeutung der Vorinkubation hingewiesen, indem gezeigt wird, daß eine 60-minütige Vorinkubation gegenüber einer Arbeitsweise ohne eine derartige Vorinkubation zu deutlich verbesserten Meßergebnissen führt. In dem o.g. maßgeblichen Artikel "Measurement of Thyrotropin Receptor Antibodies" von Bernard Rees Smith und Reginald Hall wird auf S. 417 ebenfalls die Bedeutung der Vorinkubation für ein zuverlässiges Meßverfahren hervorgehoben.

Auch als für Radiorezeptor-Assays zur Bestimmung von Anti-TSH-Rezeptor-Autoantikörpern verbesserte Reagenzien eingeführt wurden und das Meßprinzip auf Serumproben anstelle von isolierten IgG-Fraktionen ausgedehnt wurde, änderte sich am grundsätzlichen Reaktionsschema mit Vorinkubation nichts. Es kann in diesem Zusammenhang auf diejenigen der obigen Veröffentlichungen, einschließlich aller Arbeitsanleitungen, verwiesen werden, die aus den letzten zehn Jahren stammen.

Auch die Anmelderin selbst schreibt noch in ihrer Arbeitsanleitung für ihren TRAK-Assay® aus dem Jahre 1995 die übliche 15- bis 20-minütige Vorinkubation bei Raumtemperatur vor. Eine derartige Vorinkubation wird auch in allen Patenten der Anmelderin, die die Bestimmung von Anti-TSH-Rezeptor-Autoantikörpern betreffen, durchgeführt, nämlich in dem deutschen Patent DE 42 37 430, Beschreibung der Testdurchführung nach Zeile 62 von Seite 4, sowie im deutschen Patent DE 43 28 070, das ein abgewandeltes Verfahren betrifft, bei dem mit einem unmarkierten Kompetitor (bTSH) gearbeitet wird, und zwar im Versuch 3 des zuletzt genannten Patents.

Das deutsche Patent DE 42 37 430 betrifft einen Tracer in Form eines radioaktiv markierten TSH, der durch direkte Radiojodierung eines durch Aufreinigung aus crudem bovinem TSH gewonnenen TSH-Präparats, das eine biologischen Aktivität von mehr als 40 IE TSH/mg Protein aufweist und erhältlich ist durch affinitätschromatographische Reinigung von handelsüblichem crudem bovinem TSH an einer anti-TSH-Antikörper-Säule und anschließende Ionenaustausch-Chromatographie an einem schwach sauren Kationenaustauscher mit endständigen Carboxylgruppen auf der Basis eines polyamidbeschichteten Kieselgels, erhalten wurde. Dieser Tracer stellt auch den bevorzugten Tracer für das erfindungsgemäße Verfahren in seiner Modifikation als Radiorezeptor-Assay dar.

Das in der gesamten Fachwelt anerkannte Erfordernis der Durchführung einer zweistufigen Bereitung der eigentlichen Reaktionsmischung unter Anwendung einer zwischengeschalteten Vorinkubation von 15 bis 60 min stellt sich jedoch in der Routinepraxis des Diagnostiklabors als eine erhebliche Erschwernis der Testdurchführung und mögliche zusätzliche Fehlerquelle dar.

Der vorliegenden Erfindung liegt nunmehr die überraschende Feststellung zugrunde, daß die bisher einhellig als nötig angesehene Vorinkubation bei den gegenwärtigen Testverfahren unter Verwendung der im Handel befindlichen verbesserten Reagenzien gar nicht mehr erforderlich ist, sondern daß es möglich ist, auf die Vorinkubation zu verzichten und in diesem Zusammenhang auch die Folge der Pipettierschritte zu verändern, ohne daß die erhaltenen Meßergebnisse negativ beeinflußt werden.

Die folgende Erfindung betrifft somit ein an sich bekanntes Verfahren zur Bestimmung von Anti-TSH-Rezeptor-Autoantikörpern gemäß Oberbegriff von Patentanspruch 1, das dadurch gekennzeichnet ist, daß man zur Bereitung der Reaktionsmischung die Probe, das markierte TSH-Präparat und den TSH-Rezeptor im wesentlichen gleichzeitig zusammengibt und die so erhaltene Reaktionsmischung einer einstufigen Inkubation unterwirft, wobei man bei einer Pipettierung von Hand vorzugsweise so vorgeht, daß man zur Bereitung der Reaktionsmischung zuerst die Probe und das markierte TSH-Präparat zusammengibt und die Reaktion durch anschließende Zugabe des TSH-Rezeptors startet, wonach man auf an sich übliche Weise inkubiert und aufarbeitet.

Bei einer Testdurchführung mit Hilfe von Pipettierautomaten kann aber z.B. auch so vorgegangen werden, daß Probe, TSH-Rezeptor und markiertes TSH-Präparat nacheinander in der angegebenen Reihenfolge von einer Sondennadel angesaugt und gemeinsam in ein übliches Probengefäß abgegeben werden.

Das neue Inkubations- und Pipettierschema kann auch bei einem Verfahren zur Anwendung kommen, wie es im Patent DE 43 28 070 der Anmelderin beschrieben ist, bei dem man die eigentliche Konkurrenzreaktion mit einem unmarkierten TSH-Präparat durchführt und zur Bestimmung der Anwesenheit und Menge von Autoantikörpern in einer Probe anschließend die in der flüssigen Phase verbliebenen unumgesetzten TSH-Moleküle nach einem Sandwich-Verfahren bestimmt.

Diese Verfahrensvariante wird durch den nebengeordneten Anspruch 7 speziell erfaßt.

Das neuartige "Einschritt-Bestimmungsverfahren" mit nur einer einzigen gleichzeitigen Inkubation aller Reaktionsteilnehmer ergab zur eigenen Überraschung der Anmelderin bei einem entsprechenden Testversuch mit dem eigenen kommerziellen Kit für den TRAK-Assay® Ergebnisse, die denen gleichwertig waren, die mit den gleichen Reagenzien nach der üblichen Arbeitsweise erhalten wurden.

Es wird angenommen, daß durch die in der Zwischenzeit gegenüber der ursprünglichen Verfahrensführung unter Verwendung von z.B. Schilddrüsenmembranen und Immunglobulin-Fraktionen erzielten Verbesserungen sowie eine verbesserte Tracer-Herstellung die Vorinkubation überflüssig gemacht wurde, ohne daß die Fachwelt das für möglich hielt, so daß das Erfordernis der Vorinkubation noch in Veröffentlichungen der jüngeren Zeit ausdrücklich hervorgehoben wird.

Die Möglichkeit einer einstufigen Inkubation ist jedoch in der Praxis ein erheblicher Vorteil, und die Möglichkeit einer einstufigen Testdurchführung ist für den Fachmann außerordentlich überraschend.

Nachfolgend wird das erfindungsgemäße Verfahren dem traditionellen Verfahren unter Verwendung der gleichen Bestandteile des kommerziellen TRAK-Assays® der Anmelderin gegenübergestellt.

Dabei wird auf zwei Figuren Bezug genommen, die zeigen:
- Fig. 1: die Ergebnisse der Messung der gleichen 100 Patientenseren nach dem herkömmlichen und dem erfindungsgemäßen Verfahren, und
- Fig. 2: die Standardkurven für Bestimmungsverfahren unter Verwendung der Reagenzien des TRAK-Assays®, wenn einmal nach dem bekannten Verfahren und zum anderen nach dem erfindungsgemäßen Verfahren vorgegangen wurde.

### Versuchsbeschreibung

### 1. Reagenzien

Es wurden ausschließlich die üblichen Reagenzien des TRAK-Assays® der Anmelderin verwendet. Diese waren im einzelnen
A: ¹²⁵I-TSH-Präparat als Tracer, und zwar in Form eines aus Rinderhypophysen isolierten und gemäß Patent DE 42 37 430 gereinigten und radiojodierten TSH-Präparats, im Kit vorliegend in Form von Fläschchen à 40 kBq, gebrauchsfertig in 5,5 ml;
B: TSH-Rezeptor (aus Schilddrüsen vom Schwein isoliert gemäß der Arbeitsvorschrift in Patent DE 42 37 430 bzw. im o.g. Artikel von Bernard Rees Smith and Reginald Hall in: Methods in Enzymology, Vol. 74 (1981), S. 405-420), vorliegend in Fläschchen mit einem Lyophilisat, das in 1,5 ml Puffer gemäß C zu rekonstituieren ist.
C: Puffer (phosphatgepufferte Salzlösung) zur Rekonstituierung des lyophilisierten TSH-Rezeptors (Reagenz B), Fläschchen à 10,5 ml, gebrauchsfertig;
D: Fällungsreagenz, Flasche à 105 ml gebrauchsfertig, in Form einer 16%-igen Polyethylenglykol-Lösung.

Außerdem enthält der Kit noch Standards (bTSH) in Humanserum in Form von 6 gebrauchsfertigen Fläschchen à 0,5 ml zur Erstellung der Standardkurven, sowie zwei Kontrolseren in Humanserum.

### 2. Bestimmungen

Unter Verwendung der genannten Reagenzien wurden 100 Patienten-Seren vermessen, und zwar jeweils einmal unter Befolgung der in der herkömmlichen Arbeitsanleitung vorgeschriebenen Pipettier- und Inkubationsschritte, einschließlich der vorgeschriebenen Vorinkubation, sowie ein weiteres Mal nach dem erfindungsgemäßen Pipettierschema mit nur einer einzigen Inkubation.

Genauer gesagt, werden bei jeder nach dem erfindunsgemäßen Verfahren durchgeführten Messung zuerst Serumproben bzw. Standards und Kontrollseren in übliche Teströhrchen pipettiert, danach wird direkt die Tracerlösung (A) zupipettiert, und anschließend wird der rekonstituierte solubilisierte TSH-Rezeptor (B/C) zupipettiert.

Es zeigte sich, daß die Ergebnisse, die in beiden Fällen erhalten wurden, als weitgehend identisch angesehen werden können.

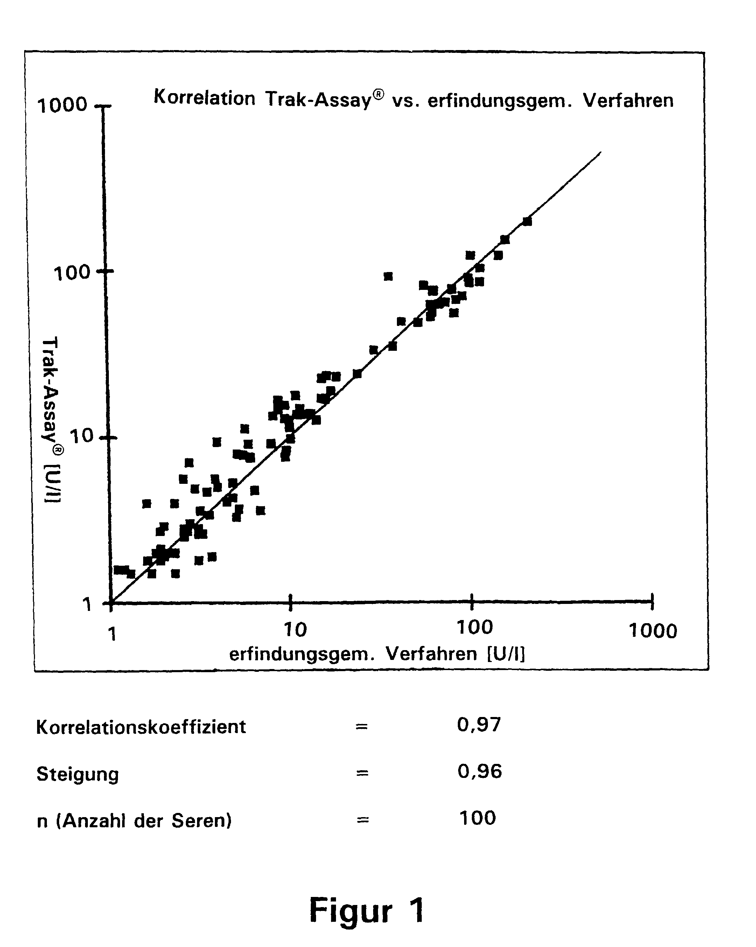

Die Ergebnisse sind graphisch in Figur 1 dargestellt.

Für die Korrelation des herkömmlichen zweistufigen Verfahrens mit dem erfindungsgemäßen einstufigen Verfahren wurde ein Korrelationskoeffizient von 0,97 bei einer Steigung von 0,96 ermittelt.

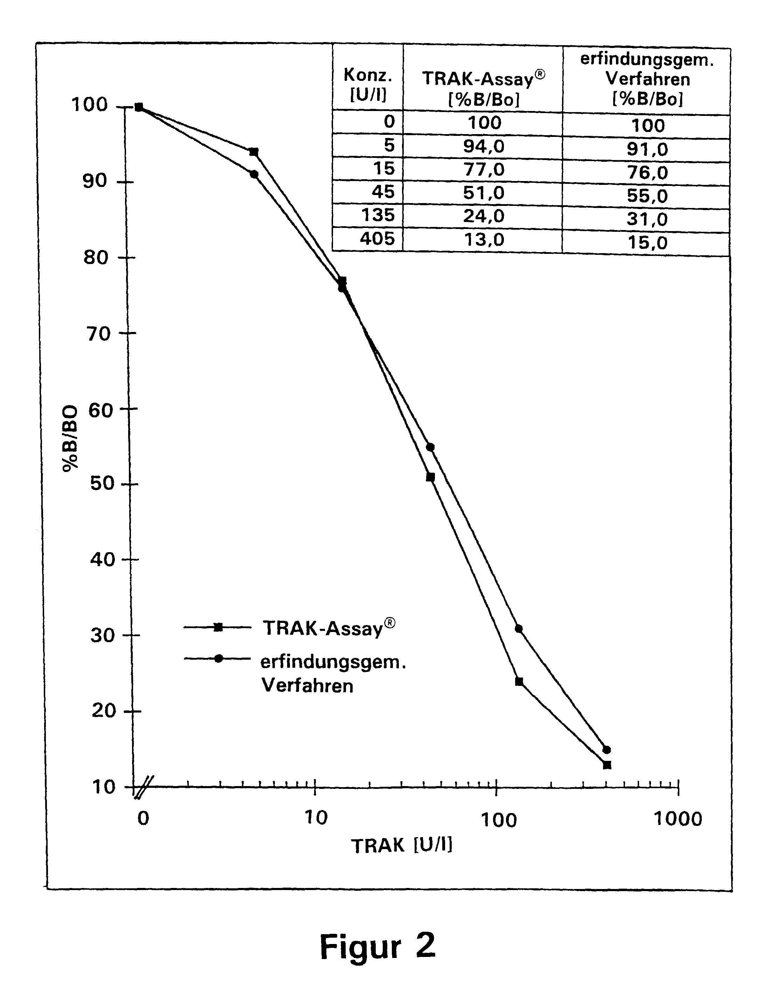

Figur 2 zeigt, daß die Standardkurven für das Bestimmungsverfahren bei herkömmlicher Testdurchführung und bei erfindungsgemäßer Testdurchführung als praktisch identisch angesehen werden können.

Auch wenn die Durchführbarkeit des erfindungsgemäßen Verfahrens unter Verwendung der konkreten Bestandteile des TRAK-Assays der Anmelderin gezeigt wurde, geht diese davon aus, daß das erfindungsgemäße Verfahren auch mit auf modifizierte Weise erhaltenen Reagenzien mit ähnlichem Ergebnis durchgeführt werden kann. So kann beispielsweise anstelle des im TRAK-Assay® verwendeten solubilisierten porcinen TSH-Rezeptors auch ein solubilisierter rekombinanter TSH-Rezeptor verwendet werden, wie er in der obigen Veröffentlichung von S. Costagliola et al. beschrieben und als äquivalent zu dem solubilisierten porcinen TSH-Rezeptor des TRAK-Assays gezeigt wurde.

Es ist ferner für das erfindungsgemäße Verfahren nicht wesentlich, auf welche Weise nach der Inkubation der Reaktionsmischung die Trennung der umgesetzten TSH-Rezeptoren von der flüssigen Reaktionsmischung erfolgt. Anstelle der üblichen PEG-Fällung können somit auch andere an sich bekannte Verfahren zur Fest-Flüssig-Trennung zur Anwendung kommen, ohne daß das auf die vorgeschaltete Umsetzung in der Reaktionsmischung einen Einfluß hätte.

## Patentansprüche

1. Verfahren zur Bestimmung von Anti-TSH-Rezeptor-Autoantikörpern in einer humanen Serum- oder Plasmaprobe, bei dem man die zu bestimmenden Autoantikörper der Probe mit einem mit einem geeigneten Label markierten TSH-Präparat um die Bindungsstellen eines solubilisierten TSH-Rezeptors konkurrieren läßt und anhand der Menge des an diesen TSH-Rezeptor gebundenen Labels auf die Menge der zu bestimmenden Autoantikörper zurückrechnet, wobei das Verfahren die Schritte umfaßt
- Inkubieren einer Reaktionsmischung, die den TSH-Rezeptor, markiertes TSH sowie die Probe enthält,
- Überführen des TSH-Rezeptors mit den daran gebundenen Anteilen an anti-TSH-Rezeptor-Autoantikörpern und markiertem TSH in eine feste Phase und deren Abtrennung von der flüssigen Phase, und
- Messung der Menge des in der festen Phase gebundenen markierten TSH anhand der für das verwendete Label typischen physikalischen oder chemischen Nachweismethode,
**dadurch gekennzeichnet,** daß man
- als TSH-Rezeptor ein durch Extraktion und Solubilisierung aus einem humanen oder tierischen Schilddrüsenmaterial gewonnenes TSH-Rezeptor-Präparat oder einen solubilisierten rekombinanten TSH-Rezeptor verwendet,
- als markiertes TSH ein radioaktiv markiertes TSH verwendet, das durch direkte Radioiodierung eines durch Aufreinigung aus crudem bovinem TSH gewonnenen TSH-Präparats, das eine biologischen Aktivität von mehr als 40 IE TSH/mg Protein aufweist und erhältlich ist durch affinitätschromatographische Reinigung von handelsüblichem crudem bovinem TSH an einer anti-TSH-Antikörper-Säule und anschließende Ionenaustausch-Chromatographie an einem schwach sauren Kationenaustauscher mit endständigen Carboxylgruppen auf der Basis eines polyamidbeschichteten Kieselgels, erhalten wurde, und
- zur Bereitung der Reaktionsmischung die Probe, das markierte TSH-Präparat und den TSH-Rezeptor im wesentlichen gleichzeitig zusammengibt und die so erhaltene Reaktionsmischung einer einstufigen Inkubation unterwirft.

2. Verfahren nach Anspruch 1, dadurch gekennzeichnet, daß man zur Bereitung der Reaktionsmischung zuerst die Probe und das markierte TSH-Präparat zusammengibt und die Reaktion durch anschließende Zugabe des TSH-Rezeptors startet.

3. Verfahren nach Anspruch 1, dadurch gekennzeichnet, daß man ein porcines TSH-Rezeptor-Präparat oder einen rekombinanten humanen TSH-Rezeptor verwendet.

4. Verfahren nach einem der vorausgehenden Ansprüche, dadurch gekennzeichnet, daß man den TSH-Rezeptor mit den daran gebundenen Anteilen an anti-TSH-Rezeptor-Autoantikörpern und markiertem TSH durch an sich bekannte PEG-Fällung und anschließende Zentrifugation von der flüssigen Phase der Reaktionsmischung abtrennt.

5. Verfahren zur Bestimmung von Anti-TSH-Rezeptor-Autoantikörpern in einer humanen Serum- oder Plasmaprobe, bei dem man
- die zu bestimmenden Autoantikörper der Probe mit einem TSH-Präparat um die Bindungsstellen eines solubilisierten TSH-Rezeptors konkurrieren läßt, indem man eine Reaktionsmischung, die den TSH-Rezeptor, das TSH-Präparat sowie die Probe enthält, unter geeigneten Bedingungen inkubiert,
- den nicht an den TSH-Rezeptor gebundenen Anteil des TSH-Präparats an eine Festphase bindet und markiert, und
- anhand der Menge der an die feste Phase gebundenen Markierung auf die Menge der zu bestimmenden Autoantikörper in der Probe zurückrechnet,
**dadurch gekennzeichnet, daß man**
- als TSH-Rezeptor ein durch Extraktion und Solubilisierung aus einem humanen oder tierischen Schilddrüsenmaterial gewonnenes TSH-Rezeptor-Präparat oder einen solubilisierten rekombinanten TSH-Rezeptor verwendet,
- als TSH ein aus crudem bovinem TSH gewonnenen TSH-Präparats verwendet, das eine biologischen Aktivität von mehr als 40 IE TSH/mg Protein aufweist und erhältlich ist durch affinitätschromatographische Reinigung von handelsüblichem crudem bovinem TSH an einer anti-TSH-Antikörper-Säule und anschließende Ionenaustausch-Chromatographie an einem schwach sauren Kationenaustauscher mit endständigen Carboxylgruppen auf der Basis eines polyamidbeschichteten Kieselgels, und
- man zur Bereitung der Reaktionsmischung die Probe, das markierte TSH-Präparat und den TSH-Rezeptor im wesentlichen gleichzeitig zusammengibt und die so erhaltene Reaktionsmischung einer einstufigen Inkubation unterwirft.

6. Verfahren nach Anspruch 5, dadurch gekennzeichnet, daß man zur Bereitung der Reaktionsmischung für die Inkubation zuerst die Probe und das markierte TSH-Präparat zusammengibt und die Reaktion durch anschließende Zugabe des TSH-Rezeptors startet.

## Claims

1. Method for the determination of anti-TSH receptor autoantibodies in a human serum or plasma sample, in which the autoantibodies to be determined in the sample are allowed to compete with a suitably labelled TSH preparation for the binding sites of a solubilized TSH receptor and the amount of autoantibodies to be determined is calculated from the amount of label bound to this TSH receptor, the method comprising the steps
- incubation of a reaction mixture which contains the TSH receptor, labelled TSH and sample,
- conversion of the TSH receptor with the fractions of anti-TSH receptor autoantibodies and labelled TSH bound thereto into a solid phase and separation of the latter from the liquid phase, and
- measurement of the amount of labelled TSH bound in the solid phase by the physical or chemical detection method typical for the label used,
characterized in that
- the TSH receptor used is a TSH receptor preparation obtained by extraction and solubilization from a human or animal thyroid material or is a solubilized recombinant TSH receptor,
- the labelled TSH used is a radiolabelled TSH which was obtained by direct radioiodination of a TSH preparation which is obtained by purification of crude bovine TSH, has a biological activity of more than 40 IU TSH/mg protein and is obtainable by purification of commercial crude bovine TSH by affinity chromatography over an anti-TSH antibody column and subsequent ion exchange chromatography over a weakly acidic cation exchanger having terminal carboxyl groups and based on a polyamide-coated silica gel, and
- for the preparation of the reaction mixture, the sample, the labelled TSH preparation and TSH receptor are combined essentially simultaneously and the reaction mixture thus obtained is subjected to a one-stage incubation.

2. Method according to Claim 1, characterized in that, for the preparation of the reaction mixture, the sample and the labelled TSH preparation are first combined and the reaction is started by subsequent addition of the TSH receptor.

3. Method according to Claim 1, characterized in that a porcine TSH receptor preparation or a recombinant human TSH receptor is used.

4. Method according to any of the preceding Claims, characterized in that the TSH receptor with the fractions of anti-TSH receptor autoantibodies and labelled TSH bound thereto is separated from the liquid phase of the reaction mixture by PEG precipitation known per se and subsequent centrifuging.

5. Method for the determination of anti-TSH receptor autoantibodies in a human serum or plasma sample, in which
- the autoantibodies to be determined in the sample are allowed to compete with a TSH preparation for the binding sites of a solubilized TSH receptor by incubating, under suitable conditions, a reaction mixture which contains the TSH receptor, the TSH preparation and the sample,
- that fraction of the TSH preparation which is not bound to the TSH receptor is bound to a solid phase and labelled, and
- the amount of autoantibodies to be determined in the sample is calculated from the amount of label bound to the solid phase,
characterized in that
- the TSH receptor used is a TSH receptor preparation obtained by extraction and solubilization from a human or animal thyroid material or is a solubilized recombinant TSH receptor,
- the TSH used is a TSH preparation which is obtained from crude bovine TSH, has a biological activity of more than 40 IU TSH/mg protein and is obtainable by purification of commercial crude bovine TSH by affinity chromatography over an anti-TSH antibody column and subsequent ion exchange chromatography over a weakly acidic cation exchanger having terminal carboxyl groups and based on a polyamide-coated silica gel, and
- for the preparation of the reaction mixture, the sample, the labelled TSH preparation and the TSH receptor are combined essentially simultaneously and the reaction mixture thus obtained is subjected to a one-stage incubation.

6. Method according to Claim 5, characterized in that, for the preparation of the reaction mixture for the incubation, the sample and the labelled TSH preparation are first combined and the reaction is started by subsequent addition of the TSH receptor.

## Revendications

1. Procédé pour la détermination d'auto-anticorps anti-récepteur de la TSH dans un échantillon de sérum ou de plasma humain, dans lequel on met en compétition les auto-anticorps à déterminer, de l'échantillon, avec une préparation de TSH marquée par un marqueur approprié, pour les sites de liaison d'un récepteur de la TSH solubilisé, et on calcule à l'aide de la quantité du marqueur lié à ce récepteur de la TSH, la quantité des auto-anticorps à déterminer, le procédé comportant les étapes consistant à:
- incuber un mélange de réaction qui contient le récepteur de la TSH, la TSH marquée ainsi que l'échantillon
- transférer le récepteur de la TSH avec les fractions d'auto-anticorps anti-récepteur de TSH et de TSH marquée qui y sont liées, dans une phase solide, et séparer cette dernière de la phase liquide, et
- mesurer la quantité de la TSH marquée liée, dans la phase solide, à l'aide du procédé de détection physique ou chimique typique pour le marqueur utilisé,
caractérisé en ce que
- comme récepteur de la TSH, on utilise une préparation de récepteur de la TSH obtenue par extraction et solubilisation d'un matériau de glande thyroïde humaine ou animale, ou un récepteur de THS recombinant solubilisé,
- comme TSH marquée, on utilise une TSH marquée radioactivement qui a été obtenue par radioiodation directe d'une préparation de TSH obtenue par purification de TSH bovine brute, qui présente une activité biologique supérieure à 40 IE TSH/mg de protéine et qui peut être obtenue par purification par chromatographie d'affinité de TSH bovine brute disponible sur le marché, sur une colonne d'anticorps anti-TSH, et ensuite chromatographie par échange d'ions sur un échangeur de cations faiblement acide présentant des groupes carboxyles terminaux, à base d'un gel de silice revêtu de polyamide, et
- pour la préparation du mélange de réaction, l'échantillon, la préparation de TSH marquée et le récepteur de la TSH sont introduits essentiellement en même temps, et le mélange de réaction ainsi obtenu est soumis à une incubation en une étape.

2. Procédé selon la revendication 1, caractérisé en ce que, pour préparer le mélange de réaction, on rassemble d'abord l'échantillon et la préparation de TSH marquée, et la réaction est amorcée par l'addition ultérieure du récepteur de la TSH.

3. Procédé selon la revendication 1, caractérisé en ce que l'on utilise une préparation de récepteur de la TSH porcin ou un récepteur de la TSH humain recombinant.

4. Procédé selon l'une des revendications précédentes, caractérisé en ce que l'on sépare le récepteur de la TSH avec les fractions d'auto-anticorps anti-récepteur de la TSH et de TSH marquée qui y sont liées, par une précipitation par PEG, en soi connue, et ensuite séparation de la phase liquide du mélange de réaction par centrifugation.

5. Procédé pour la détermination d'auto-anticorps anti-récepteur de la TSH dans un échantillon de sérum ou de plasma humain, dans lequel:
- les auto-anticorps à déterminer dans l'échantillon sont mis en compétition avec une préparation de TSH pour les sites de liaison d'un récepteur de la TSH solubilisé, en incubant un mélange de réaction qui contient le récepteur de la TSH, la préparation de TSH ainsi que l'échantillon, dans des conditions appropriées,
- la fraction de la préparation de TSH non liée au récepteur de la TSH est liée à une phase solide et marquée, et
- à partir de la quantité de l'élément marqué lié à la phase solide, on calcule la quantité de l'auto-anticorps à déterminer dans l'échantillon,
caractérisé en ce que
- comme récepteur de la TSH, on utilise une préparation de récepteur de la TSH obtenue par extraction et solubilisation d'un matériau de glande thyroïde humaine ou animale, ou un récepteur de la TSH recombinant solubilisé,
- comme TSH, on utilise une préparation de TSH obtenue à partir de TSH bovine brute, qui présente une activité biologique supérieure à 40 IE TSH/mg de protéine, et qui peut être obtenue par purification par chromatographie d'affinité de TSH bovine brute disponible sur le marché, sur une colonne d'anticorps anti-TSH, et ensuite chromatographie par échange d'ions sur un échangeur de cations faiblement acide présentant des groupes carboxyles terminaux, à base d'un gel de silice revêtu de polyamide, et
- pour la préparation du mélange de réaction, l'échantillon, la préparation de TSH marquée et le récepteur de la TSH sont introduits essentiellement en même temps, et le mélange de réaction ainsi obtenu est soumis à une incubation en une étape.

6. Procédé selon la revendication 5, caractérisé en ce que, pour la préparation du mélange de réaction en vue de l'incubation, on combine d'abord l'échantillon et la préparation de TSH marquée, et on amorce la réaction par l'addition ultérieure du récepteur de la TSH.
